# DEMANDE DE BREVET EUROPEEN

(11) **EP 1 685 876 A1**
(43) Date de publication de la demande: **02.08.2006**
(21) Numéro de dépôt: 06000058.5
(22) Date de dépôt: 03.01.2006
(51) Int. Cl.: A61Q 19/10, A61K 8/02, A61K 8/368, A61K 9/14, A61Q 5/02, A61K 31/60, A61K 8/73, A61P 17/10

(54) **Composition cosmétique nettoyante sous forme solide**

(30) Priorité: 31.01.2005 FR 0550264
(71) Demandeur: L'ORÉAL, 75008 Paris (FR)
(72) Inventeur: Calduk, Agnès, 92260 Fontenay-aux-roses (FR)
(74) Mandataire: Rasson, Catherine

(57) **Abrégé**

L'invention se rapporte à une composition de nettoyage de la peau, se présentant sous forme d'un solide et comportant, dans un milieu anhydre, au moins un actif kératolytique choisi parmi l'acide salicylique et ses dérivés, au moins un tensioactif moussant pulvérulent, et au moins un polymère hydrosoluble, la quantité de polymère hydrosoluble pouvant aller jusqu'à 10 % du poids total de la composition.

La composition se présente sous forme de poudre ou de tablette et elle peut être incorporée dans une éponge.

La composition peut être utilisée notamment pour le nettoyage des peaux grasses à tendance acnéique.

## Description

L'invention se rapporte à une composition de nettoyage de la peau se présentant sous forme d'un solide et notamment sous forme d'une poudre, comportant de l'acide salicylique ou un de ses dérivés, au moins un tensioactif moussant pulvérulent et au moins un polymère hydrosoluble, et à son utilisation pour le nettoyage de la peau aussi bien du corps que du visage, y compris autour des yeux.

Le nettoyage de la peau est très important pour le soin du visage. Il doit être le plus performant possible car les résidus gras tels que l'excès de sébum, les restes des produits cosmétiques utilisés quotidiennement et les produits de maquillage notamment les produits « waterproof » résistants à l'eau, s'accumulent dans les replis cutanés et peuvent obstruer les pores de la peau et entraîner l'apparition de boutons.

Il est connu d'incorporer dans les compositions de nettoyage de la peau, des actifs kératolytiques et notamment de l'acide salicylique ou un dérivé d'acide salicylique, ces actifs permettant de nettoyer la peau en profondeur et d'avoir une action sur les comédons dans le cas des peaux à tendance acnéiques. Les compositions nettoyantes se présentent le plus souvent sous forme de compositions aqueuses plus ou moins liquides contenant des tensioactifs moussants.

Toutefois, lorsqu'une telle composition contient un pourcentage élevé de tensioactifs moussants (par exemple plus de % de tensioactifs), il est difficile d'y incorporer un pourcentage important d'actif kératolytique et notamment d'acide salicylique, en raison de problème de solubilisation ou de pH, car ces actifs, bien qu'ils restent eux-mêmes stables, peuvent déstabiliser la composition aqueuse les contenant et entraîner une chute de la viscosité, et parfois même provoquer un jaunissement de la composition les contenant.

En outre, il est difficile d'avoir une composition moussante contenant une forte quantité de tensioactif et de l'acide salicylique, qui soit en même temps bien tolérée et qui soit confortable.

Il subsiste donc le besoin d'une composition contenant un pourcentage élevé de tensioactifs et pouvant contenir de l'acide salicylique ou un de ses dérivés, sans que cela ne pose de problème de formulation ni d'innocuité..

La demanderesse a trouvé qu'il était possible d'obtenir une composition de nettoyage surmontant les inconvénients de l'art antérieur, en préparant une composition de nettoyage anhydre solide contenant des tensioactifs anioniques pulvérulents et au moins un polymère hydrosoluble.

La présente invention a pour objet une composition de nettoyage de la peau, se présentant sous forme solide et comportant, dans un milieu anhydre physiologiquement acceptable, au moins un actif choisi parmi l'acide salicylique et ses dérivés, au moins un tensioactif anionique moussant pulvérulent et au moins un polymère hydrosoluble, la quantité de polymère hydrosoluble pouvant aller jusqu'à 10 % du poids total de la composition.

On entend par « composition sous forme solide » une composition qui peut se présenter sous forme d'une tablette (ou comprimé) (solide d'une dimension allant jusqu'à environ 4 cm) ou d'une poudre, une poudre étant un solide finement divisé en particules (particules pouvant avoir une dimension allant par exemple de 50 à 600 µm), par opposition à une composition sous forme de crème ou de gel qui sont des compositions souples.

Par ailleurs, on entend par « pulvérulent », un composé ou une composition se présentant sous forme de poudre (constituée de particules ou grains).

La composition selon l'invention est préparée par simple mélange des constituants qui sont tous pulvérulents ; elle est donc facile à fabriquer. En outre, elle a une excellente solubilité dans l'eau et elle a de bonnes propriétés moussantes, tout en étant confortable et bien tolérée comme le montre le test présenté plus loin.

Par ailleurs, on entend par « milieu physiologiquement acceptable » un milieu compatible avec les matières kératiniques, et notamment la peau.

La composition de l'invention peut constituer une composition cosmétique ou dermatologique.

### Acide salicylique et dérivés

La composition de l'invention peut comprendre de l'acide salicylique et/ou un ou plusieurs dérivés de l'acide salicylique, composés se présentant sous forme de poudre.

Comme dérivés de l'acide salicylique, on peut utiliser tout dérivé susceptible d'être utilisé dans une composition cosmétique et/ou dermatologique, et notamment les dérivés d'acide salicylique de formule (I) ou un sel d'un tel dérivé : 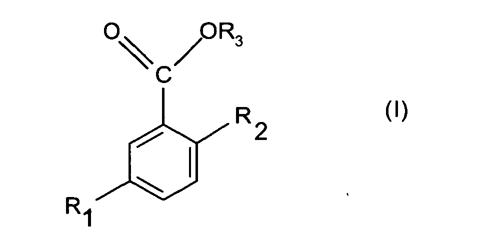 dans laquelle :
R₁ représente l'hydrogène ou une chaîne aliphatique, alcoxy, ester ou cétoxy, saturée, linéaire, ramifiée ou cyclisée, une chaîne insaturée portant une ou plusieurs doubles liaisons conjuguées ou non, ces chaînes comportant de 1 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;
R₂ représente un groupement hydroxyle ou une fonction ester de formule (II) : 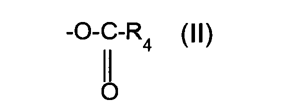 où R₄ représente un groupement aliphatique saturé ou un groupement alcényle ayant de 1 à 18 atomes de carbone ;
R₃ représente l'hydrogène ou une chaîne linéaire ou ramifiée, saturée ou insaturée, ayant de 2 à 30 atomes de carbone, comportant éventuellement un ou plusieurs substituants. La chaîne peut être choisie en particulier parmi les radicaux alkyle et alcényle comportant de 2 à 30 atomes de carbone, éventuellement substitués. Le substituant peut être en particulier un radical hydroxylé.

Quand R₃ est l'hydrogène, on peut aussi utiliser des sels des acides de formule (I), et en particulier des sels obtenus par salification avec une base.

Comme base susceptible de salifier les dérivés de l'acide salicylique de formule (I), on peut citer les bases minérales comme les hydroxydes de métaux alcalins (hydroxydes de sodium et de potassium) ou les hydroxydes d'ammonium ou mieux encore les bases organiques telles que les amines organiques primaires, secondaires, tertiaires ou cycliques et plus spécialement les acides aminés. A titre d'exemple de bases, on peut citer la glycine, la lysine, l'arginine, la taurine, l'histidine, l'alanine, la valine, la cystéïne, la trihydroxy-méthylaminométhane (TRISTA), la triéthanolamine.

Selon un mode particulier de réalisation de l'invention, on utilise dans la composition de l'invention, comme dérivé de formule (I), ceux où le radical R₁ comporte au moins 4 atomes de carbone. Il est par exemple formé d'un radical alkyle ou alcoxy linéaire saturé ayant de 4 à 11 atomes de carbone.

Comme dérivés de formule (I) dans laquelle R₁ est formé d'un radical alkyle ou alcoxy linéaire saturé ayant de 4 à 11 atomes de carbone, R₂ est un hydroxyle et R₃ est l'hydrogène, on peut citer notamment les acides n-octanoyl-5-salicylique (nom CTFA : Capryloyl Salicylic Acid), n-décanoyl-5-salicylique, n-dodécanoyl-5-salicylique, n-octyl-5-salicylique, n-heptyloxy-5-salicylique, 5-tert-octylsalicylique, 5-butoxysalicylique, 5-éthoxysalicylique, 5-méthoxysalicylique, 5-propoxysalicylique, 5-méthylsalicylique, 5-éthylsalicylique, 5-propylsalicylique, et leurs mélanges, ces acides étant éventuellement salifiés par une base. On peut également utiliser les composés décrits dans le document EP-A-570230.

Quand R₁ représente l'hydrogène et R₂ un groupement hydroxyle, le dérivé de formule (I) est un ester d'acide salicylique. Il s'agit de préférence d'esters d'alcools gras tels que les esters d'alcools dodécylique, hexadécylique, stéarylique, cétylique, myristylique, linoléylique, octylique, oléique, tridécylique, ou également d'esters d'alcools butylique, propylique, éthylique, ou encore les esters de polyols tels que les esters de propylène glycol, de butylène glycol, d'éthylène glycol ou de glycérol, ou des mélanges de ces esters. Il peut s'agir notamment du salicylate de cétyle, du salicylate de dodécyle et du salicylate de tridécyle.

Selon un mode préféré de réalisation de l'invention, l'actif est l'acide salicylique.

La quantité d'actif choisi parmi l'acide salicylique et ses dérivés peut aller par exemple de 0,1 à 30 % en poids, de préférence de 0,5 à 10 % en poids et mieux de 0,5 à 5 % en poids par apport au poids total de la composition.

### Tensioactif anionique moussant

La composition selon l'invention contient au moins un tensioactif anionique moussant pulvérulent, c'est-à-dire sous forme de poudre. On peut aussi éventuellement utiliser des tensioactifs sous forme de pâte. La quantité de tensioactif(s) moussant(s) peut aller par exemple de 5 à 99,9 % en poids, de préférence de 10 à 99 % en poids et mieux de 20 à 98 % par rapport au poids total de la composition.

Selon un mode préféré de réalisation de l'invention, la composition contient comme tensioactif anionique moussant, au moins un sel d'acides gras. Les sels d'acide gras qui constituent les savons sont dérivés d'un acide gras ayant une chaîne alkyle comportant de 6 à 22 atomes de carbone, de préférence de 8 à 18 atomes de carbone. Il s'agit notamment de sels obtenus par neutralisation d'un acide gras par une base organique ou minérale telle que la potasse, la soude, la triéthanolamine, la N-methylglucamine, la lysine et l'arginine. Comme sels d'acides gras (savons), on peut citer notamment les sels acalins et par exemple les sels de potassium ou de sodium des acides laurique, myristique, palmitique, stéarique (laurate, myristate, palmitate et stéarate de potassium ou de sodium), en particulier le laurate de potassium et le myristate de potassium, et plus particulièrement le myristate de potassium qui est de préférence utilisé comme savon dans le cadre de la présente invention.

On peut aussi utiliser comme tensioactifs anioniques pulvérulents, les sels alcalins de N-acylaminoacides, les alkylsulfates et alkylethersulfates, les sulfonates, et leurs mélanges.

Comme sels alcalins de N-acylaminoacides, on peut citer notamment les sarcosinates, les alaninates, les glutamates, les aspartates et leurs mélanges,

Les tensioactifs en poudre ou se présentant sous forme de pâte peuvent être choisis par exemple parmi le Lauroyl sarcosinate de sodium comme le produit commercialisé sous la dénomination Sarkosyl NL 97 par la société Ciba ; le myristoyl sarcosinate de sodium comme le produit commercialisé sous la dénomination Nikkol Sarcosinate MN par la société Nikkol, le palmitoyl sarcosinate de sodium comme le produit commercialisé sous la dénomination Nikkol Sarcosinate PN par la société Nikkol, le lauryl sulfate de sodium comme le produit commercialisé sous la dénomination Empicol LZ D par la société Allbright & Wilson ou sous la dénomination Tensopol USP97 par la société Tensachem ; le lauroyl glutamate de sodium comme le produit commercialisé sous la dénomination Amisoft LS 11 par la société Ajinomoto ; le myristoyl glutamate monosodique comme le produit commercialisé sous la dénomination Acylglutamate MS 11 par la société Ajinomoto ; le mélange de laureth sulfate de sodium et de silice, commercialisé sous la dénomination Texapon KE 2713 par la société Cognis ; le disodium cocamido MEA-sulfosuccinate comme le produit commercialisé sous la dénomination Mackanate CM 100 par la société Mac Intyre ; le methyl cocoyl taurate de sodium, comme le produit commercialisé sous la dénomination Tauranol WSP par la société Finetex ; le decyl d-galactoside uronate de sodium comme le produit commercialisé sous la dénomination Decyl d-galactoside uronate de sodium par la société Ard-Soliance ; le lauroyl methyl beta-alanine (forme acide) commercialisé sous la dénomination LMA-H par la société Mitsui Toatsu ; la n-lauroyl-n-hydroxyethyl-beta-alanine commercialisée sous la dénomination LHEA par la société Mitsui Toatsu ; le cocoyl glycinate de sodium commercialisé sous la dénomination Amilite GCS-11(F) par la société Ajinomoto ; le cocoyl isethionate de sodium comme le produit commercialisé sous la dénomination Jordapon CI P par la société BASF ; le sel de sodium du methyltaurate d'huile de palmiste comme le produit commercialisé sous la dénomination Hostapon CT PATE® par la société Clariant ; le N-cocoyl N-methyltaurate de sodium comme le produit commercialisé sous la dénomination Hostapon LT-SF® par la société Clariant ou Nikkol CMT-30-T® par la société Nikkol ; le palmitoyl methyltaurate de sodium comme le produit commercialisé sous la dénomination Nikkol PMT® par la société Nikkol ; le lauryl sulfoacétate de sodium, comme le produit commercialisé sous la dénomination Lathanol LAL poudre par la société Stepan ; le myristate de potassium comme le produit commercialisé sous la dénomination Myristate de potassium (DUB MK) par la société Stearinerie Dubois; le laurate de potassium comme le produit commercialisé sous la dénomination Laurate de potassium (DUB LK) par la société Stearinerie Dubois, et le laurate de sucrose comme le produit commercialisé sous la dénomination Grilloten LSE 87 par la société Degussa.

Selon un mode préféré de réalisation de l'invention, la composition contient comme tensioactifs anioniques moussants, un ou plusieurs sels d'acide gras, seuls ou en mélange avec un ou plusieurs sels d'iséthionate tels que le cocoyl isethionate de sodium. Selon un mode particulièrement préféré de réalisation de l'invention, la composition contient comme tensioactifs anioniques moussants, du myristate de potassium et du cocoyl isethionate de sodium.

La composition peut en outre éventuellement contenir un ou plusieurs tensioactifs non ioniques ou amphotères, comme par exemple les esters de sucre ; les alkyl polyglucosides (APG), de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de saccharide ; les bétaïnes. Comme tensioactifs non ioniques ou amphotères, on peut citer en particulier la cocamidopropylbetaine comme le produit commercialisé sous la dénomination Tegobetain CK D par la société Degussa et le laurate de sucrose comme le produit commercialisé sous la dénomination Grilloten LSE 87 par la société Degussa.

### Polymère hydrosoluble

La composition selon l'invention comprend au moins un polymère hydrosoluble. Elle peut en contenir plusieurs, la quantité totale de polymères hydrosolubles pouvant aller jusqu'à 10 % du poids total de la composition.

On entend par « polymère hydrosoluble », un polymère qui gonfle ou se solubilise dans l'eau à la température ambiante (température d'environ 20 à 25°C).

Ce polymère hydrosoluble peut être notamment choisi parmi les polysaccharides dont les gommes, les polymères synthétiques, les dérivés cellulosiques et leurs mélanges.

A titre d'exemple de polymères hydrosolubles utilisables dans l'invention, on peut citer les gommes de guar, de xanthane, de carraghénane, de cellulose, de sclerotium et les dérivés de ces gommes ; les hydroxyalkylcelluloses et les carboxycelluloses de sodium; les polyacrylamides et les copolymères d'acrylamides ; la gélatine ; l'agar-agar ; les polymères carboxyvinyliques tels que les produits commercialisés sous les dénominations carbopol par la société Noveon (nom CTFA : carbomer), les polymères carboxyvinyliques modifiés et notamment les copolymères acrylate/C₁₀-C₃₀-alkylacrylate tels que les produits commercialisés sous les dénominations Pemulen TR1 ou TR2 ou CARBOPOL 1382 par la société Noveon (nom CTFA : Acrylates/C10-30 Alkyl acrylate Crosspolymer).

Selon un mode préféré de réalisation de l'invention, la composition contient un polymère hydrosoluble pulvérulent, plus particulièrement une gomme et en particulier de la gomme de xanthane.

La quantité de polymère(s) hydrosoluble(s) dans la composition de l'invention peut aller jusqu'à 10 % en poids par rapport au poids total de la composition. Cette quantité peut aller par exemple de 0,01 à 10 % en poids, de préférence de 0,1 à 10 % mieux de 0,5 à 10 % en poids, et encore mieux de 1 à 5 % en poids par rapport au poids total de la composition.

### Additifs

La composition de l'invention peut contenir un ou plusieurs additifs du moment que ceux-ci sont anhydres ou sous forme solide (poudre), notamment choisis parmi ceux généralement utilisés dans les domaines cosmétique et dermatologique, tels que, par exemple, les séquestrants, les parfums, les antioxydants, les actifs autres que l'acide salicylique et ses dérivés, les conservateurs, les matières colorantes (comme les pigments et les colorants hydrophiles) et les charges minérales et/ou les charges organiques telles que l'amidon modifié comme celui commercialisé sous la dénomination Dry Flo par la société National Starch. Quand ils sont présents, ces additifs peuvent être en une quantité allant de 0,1 à 50 % et de préférence de 0,5 et 50 % du poids total de la composition.

La composition selon l'invention peut contenir aussi une ou plusieurs huiles ou actif huileux, mais en une quantité inférieure ou égale à 5 % du poids total de la composition.

Bien entendu, l'homme de l'art veillera à choisir ce ou ces éventuels additifs et/ou leurs quantités de manière telle que les propriétés avantageuses attachées intrinsèquement à la composition conforme à l'invention ne soient pas, ou substantiellement pas, altérées par la ou les adjonctions envisagées.

Comme actifs, on peut citer par exemple l'acide ascorbique (vitamine C).

Selon un mode particulier de réalisation de l'invention, la composition est exempte de polyol (glycérine, glycols) ou en contient une quantité inférieure à 5 % du poids total de la composition.

La composition de l'invention est préparée par simple mélange des constituants dans une cuve de mélange, en évitant tout contact avec de l'eau ou de l'humidité.

De façon avantageuse, la composition de l'invention comporte un milieu physiologiquement acceptable, c'est-à-dire un milieu compatible avec les matières kératiniques et notamment avec la peau et/ou les cheveux. Elle peut constituer notamment une composition cosmétique ou dermatologique.

La composition de l'invention donne lieu à la formation de mousse en présence d'eau et présente notamment une bonne efficacité pour le nettoyage de la peau.

Aussi, la présente invention a aussi pour objet l'utilisation cosmétique de la composition selon l'invention pour nettoyer la peau ou les cheveux.

La présente invention a aussi pour objet un procédé cosmétique de nettoyage de la peau, consistant à mouiller la composition avec de l'eau, à la passer sur la peau et à rincer la peau.

La composition selon l'invention peut aussi être utilisée pour le traitement des peaux grasses à tendance acnéique, peaux qui présentent généralement des imperfections cutanées, des pores dilatés, une texture inhomogène de la peau et des rougeurs.

Ainsi, la présente invention a encore pour objet un procédé de traitement cosmétique d'une peau à tendance acnéique, comprenant l'application topique sur ladite peau de la composition précitée.

Par ailleurs, la composition selon l'invention peut aussi être utilisée pour le nettoyage d'une peau comportant de l'acné avant le soin.

L'invention a donc aussi pour objet l'utilisation de la composition telle que définie ci-dessus pour la préparation d'une composition dermatologique destinée au traitement de l'acné.

Selon un mode particulier de réalisation de l'invention, la composition de l'invention, qu'elle soit sous forme de poudre ou de comprimé ou de tablette, est incorporée dans une éponge ou un article analogue présentant une cavité, en présentation unidose. Pour l'utiliser, on mouille l'éponge, puis on la passe sur le visage et on rince.

La présente invention a aussi pour objet une éponge contenant une composition selon l'invention, et à son utilisation pour le nettoyage de la peau.

On donne, ci-après, des exemples de compositions conformes à l'invention. Les quantités sont données en pourcentage pondéral.

### Exemple 1 : composition de nettoyage

- Cocoyl iséthionate de sodium 45,5 %
- Gomme de xanthane 3 %
- Myristate de potassium 45,5 %
- Acide salicylique 2 %
- Parfum 4 %

La composition a été obtenue par mélange des constituants qui sont tous en poudre. Elle se présentait sous forme d'une poudre. Une quantité de 0,7 g de cette poudre a été introduite dans une éponge en ouate de forme ronde ayant un diamètre d'environ 70 mm.

On utilise cette éponge en la mouillant à l'eau, en la passant sur la peau à nettoyer et en rinçant.

Un test réalisé sur 42 femmes pendant 4 semaines d'application avec une application par jour a montré que :
- pour 100% des femmes, la mousse était abondante ;
- pour 95% des femmes, l'éponge nettoyait en profondeur ;
- pour 97% des femmes, le nettoyage laissait la peau nette et purifiée ;
- pour 69% des femmes, les pores étaient moins visibles ;
- pour 88% des femmes, les imperfections étaient gommées ;
- pour 95% des femmes, la composition était facile à rincer ;
- pour 74% des femmes, le nettoyage laissait une peau confortable (sans tiraillement).

### Exemple 2 : composition de nettoyage

- Cocoyl iséthionate de sodium 45,5 %
- Gomme de xanthane 3 %
- Aluminium starch octenyl succinate (charge) 45,5 %
- Acide salicylique 2 %
- Parfum 4 %

La composition a été préparée comme dans l'exemple 1. On a obtenu une composition sous forme de poudre. Une quantité de 1 g de cette poudre a été comprimée sous forme de tablette et a été introduite dans une éponge qui a été utilisée comme l'éponge de l'exemple 1.

## Revendications

1. Composition de nettoyage de la peau, se présentant sous forme d'un solide et comportant, dans un milieu anhydre, au moins un actif choisi parmi l'acide salicylique et ses dérivés, au moins un tensioactif anionique moussant pulvérulent et au moins un polymère hydrosoluble, la quantité de polymère hydrosoluble pouvant aller jusqu'à 10 % du poids total de la composition.

2. Composition selon la revendication 1, **caractérisée en ce que** la quantité d'actif(s) choisi(s) parmi l'acide salicylique et ses dérivés va de 0,1 à 30 % en poids par rapport au poids total de la composition.

3. Composition selon la revendication 1 ou 2, **caractérisée en ce que** l'actif est choisi parmi les dérivés d'acide salicylique de formule (I) ou un sel d'un tel dérivé : 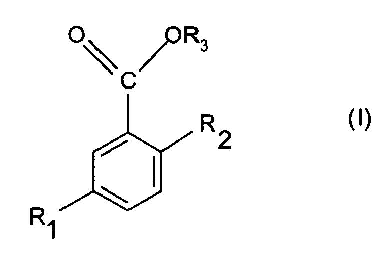 dans laquelle :
R₁ représente l'hydrogène ou une chaîne aliphatique, alcoxy, ester ou cétoxy, saturée, linéaire, ramifiée ou cyclisée, une chaîne insaturée portant une ou plusieurs doubles liaisons conjuguées ou non, ces chaînes comportant de 1 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;
R₂ représente un groupement hydroxyle ou une fonction ester de formule (II) : 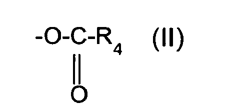 où R₄ représente un groupement aliphatique saturé ou un groupement alcényle ayant de 1 à 18 atomes de carbone ;
R₃ représente l'hydrogène ou une chaîne linéaire ou ramifiée, saturée ou insaturée, ayant de 2 à 30 atomes de carbone, comportant éventuellement un ou plusieurs substituants.

4. Composition selon la revendication précédente, **caractérisée en ce que** le dérivé d'acide salicylique est choisi parmi les acides n-octanoyl-5-salicylique, n-décanoyl-5-salicylique, n-dodécanoyl-5-salicylique, n-octyl-5-salicylique, n-heptyl-oxy-5-salicylique, 5-tert-octylsalicylique, 5-butoxysalicylique, 5-éthoxysalicylique, 5-méthoxysalicylique, 5-propoxysalicylique, 5-méthylsalicylique, 5-éthylsalicylique, 5-propylsalicylique, et leurs mélanges.

5. Composition l'une quelconque des revendications précédentes, **caractérisée en ce que** la quantité de tensioactif(s) anionique(s) moussant(s) va de 5 à 99,9 % en poids par rapport au poids total de la composition.

6. Composition selon l'une quelconque des revendications précédentes, **caractérisée en ce qu'**elle contient comme tensioactif anionique, un ou plusieurs sels alcalins d'acides gras.

7. Composition selon l'une quelconque des revendications précédentes, **caractérisée en ce qu'**elle contient comme tensioactif anionique, un ou plusieurs sels alcalins d'acides gras et un ou plusieurs sels d'isethionate.

8. Composition selon l'une quelconque des revendications précédentes, **caractérisée en ce que** le polymère hydrosoluble est choisi parmi les polysaccharides, les polymères synthétiques, les celluloses, et leurs mélanges.

9. Composition selon la revendication précédente, **caractérisée en ce que** le polymère hydrosoluble est la gomme de xanthane.

10. Composition selon l'une quelconque des revendications précédentes, **caractérisée en ce que** la quantité de polymère(s) va 0,01 à 10 % du poids total de la composition.

11. Composition selon l'une quelconque des revendications précédentes, **caractérisée en ce qu'**elle se présente sous forme de tablette ou de poudre.

12. Eponge contenant une composition selon l'une quelconque des revendications 1 à 11.

13. Utilisation cosmétique de la composition selon l'une quelconque des revendications 1 à 11 ou de l'éponge selon la revendication 12, pour nettoyer la peau ou les cheveux.

14. Procédé cosmétique de nettoyage, **caractérisé en ce qu'**il consiste à mouiller la composition selon l'une quelconque des revendications 1 à 11 ou de l'éponge selon la revendication 12, avec de l'eau, à la passer sur la peau et à rincer la peau.

15. Procédé de traitement cosmétique d'une peau à tendance acnéique, comprenant l'application topique sur ladite peau, de la composition selon l'une quelconque des revendications 1 à 11 ou de l'éponge selon la revendication 12.

16. Utilisation de la composition selon l'une quelconque des revendications 1 à 11 ou de l'éponge selon la revendication 12, pour la préparation d'une composition dermatologique destinée au traitement de l'acné.
